# EUROPEAN PATENT APPLICATION

(11) **EP 4 190 905 A1**
(43) Date of publication of application: **07.06.2023**
(21) Application number: 21306680.6
(22) Date of filing: 01.12.2021
(51) Int. Cl.: C12N 15/82

(54) **METHOD FOR THE PURIFICATION OF RECOMBINANT PROTEINS USING OIL BODY PROTEINS**

(71) Applicant: Core Biogenesis, 67400 Illkirch-Graffenstaden (FR)
(72) Inventor: MEZIADI, Chouaïb, 67400 ILLKIRCH-GRAFFENSTADEN (FR); MOLONEY, Maurice M., 67400 ILLKIRCH-GRAFFENSTADEN (FR); AGUILAR-SALVADOR, Daniel-Isui, 67400 ILLKIRCH-GRAFFENSTADEN (FR); NEY, Michel, 67400 ILLKIRCH-GRAFFENSTADEN (FR); RAGE, Emile, 67400 ILLKIRCH-GRAFFENSTADEN (FR)
(74) Representative: Atout PI Laplace

(57) **Abstract**

The invention relates to a method for the purification of recombinant proteins. Particularly, the present invention relates to a method for the purification of recombinant proteins produced by a first genetically engineered organism by mixing the first genetically engineered organism or part thereof with oil body proteins which are not obtained from the first genetically engineered organism.

## Description

### FIELD OF THE INVENTION

The present invention relates to a method for the purification of recombinant proteins. Particularly, the present invention relates to a method for the purification of recombinant proteins produced by a first genetically engineered organism by mixing the first genetically engineered organism or part thereof with oil body proteins which are not obtained from the first genetically engineered organism.

### BACKGROUND PRIOR ART

A recombinant protein is a protein produced by cells whose DNA has been modified by genetic engineering. Recombinant proteins can be molecules of pharmaceutical, therapeutic, cosmetic interest, molecules used in research and development or in the field of agroindustry. Several recombinant protein expression systems using bacteria, yeast, mammalian cells, or other organisms, have been developed. These conventional cell culture-based systems are useful but limited by high cost, low efficiency or low yield. Notably, these systems have an expensive extraction process or, for the most economically attractive ones, do not allow a step of maturation of the recombinant protein which is essential so as to render the recombinant protein functional.

Plants as recombinant protein production hosts have been considered as a viable option. Indeed, they provide a valuable alternative to fermentation-based systems for the production of recombinant proteins, having numerous advantages. Notably, transient expression in plant leaves is one of the most promising technologies for the production of recombinant proteins of interest. In particular, such technology allows for production of mature proteins, i.e. which have been subjected to successful post-translational modifications. However, the extraction of these recombinant proteins relies mainly on the use of tags, making this method undesirable, especially for regulatory reasons and expensive. Also, other proteins may interfere with the purification method, making the extraction process less efficient.

US patent 7,547,821 B2 discloses a method for the expression of insulin in plant seeds comprising the introduction into a plant cell of a chimeric nucleic acid construct which includes a first sequence comprising a seed specific promoter, a second sequence encoding a signal peptide for the secretory pathway and an insulin polypeptide and a third sequence encoding a single chain antibody (scFv) having specificity for an oil body protein. However, such a method does not allow an easy and rapid protein expression as the method requires growing the plant cell into a mature plant capable of setting seed and obtaining substantially pure insulin from said seeds. Notably, such a method takes multiple months in order to obtain the recombinant protein while the demand for affordable insulin, or other recombinant proteins, is escalating.

There is therefore a need for a new method or system which allows production a sufficient amount of recombinant protein in a relatively short time while reducing the cost of goods of the recombinant protein to an affordable price commensurate with the need.

The invention seeks to overcome the aforementioned drawbacks of the prior art as it aims to provide such a method which allows for easy and rapid protein expression and recovery.

### SUMMARY OF THE INVENTION

To this effect the invention discloses a method for the purification of recombinant proteins produced by a first genetically engineered organism, the method comprising the following steps: providing the first genetically engineered organism expressing the recombinant proteins or a part thereof; mixing the first genetically engineered organism or part thereof with oil body proteins which are not obtained from the first engineered organism, wherein the recombinant proteins and/or the oil body proteins are fused with an affinity ligand which allows forming complexes comprising the oil body proteins and the recombinant proteins; recovering the complexes comprising the oil body proteins and the recombinant proteins by flotation of the mix.

Advantageously, the first genetically engineered organism is a genetically engineered plant.

Advantageously, providing the first genetically engineered plant expressing the recombinant protein or a part thereof consists in providing the leaves of the genetically engineered plant.

Advantageously, the genetically engineered plant expressing the recombinant proteins has been engineered by introducing a nucleic construct comprising the coding sequence of the recombinant proteins via agroinfiltration.

Advantageously, the genetically engineered plant or part thereof expressing the recombinant proteins comprises at least one inactivated gene involved in the Transcriptional Gene Silencing (TGS) and/or at least one inactivated gene involved in the Post Transcriptional Gene Silencing (PTGS) mechanism.

Advantageously, the oil body proteins are selected among a list comprising caveolin, oleosin, caleosin, steroleosin, perilipin or combinations thereof.

Advantageously, the oil body proteins are oleosin.

Advantageously, the recombinant proteins are selected among a list comprising insulin, growth factors, therapeutic antibodies, antigens, structural and bioactive peptides or combinations thereof.

Advantageously, the affinity ligand which allows forming complexes comprising the oil body proteins and the recombinant proteins is selected among: a shark affinity ligand, an artificial affinity ligand derived through phage display or bioinformatics or protein predictive models, or combinations thereof.

Advantageously, the affinity ligand which is able to form complexes comprising the oil body proteins and the recombinant proteins is selected among: an antibody or fragment thereof, a single-chain variable fragment (scFv), a sdAb (single-domain antibody), or combinations thereof.

Advantageously, the affinity ligand is a scFv.

Advantageously, the step of mixing comprises mixing the first genetically engineered organism or part thereof with an oilseed plant comprising oil body proteins, preferably the seeds of an oilseed plant comprising oil body proteins.

Advantageously, the affinity ligand fused with the recombinant proteins and/or with the oil body proteins contains a cleavage site.

Advantageously, the method further comprises a step of purifying the recombinant proteins from the complexes.

Advantageously, purifying the recombinant proteins from the complexes includes modifying the pH to cleave the affinity ligand from the complexes.

Further characteristics, details and advantages of the invention will emerge from the description made with reference to the annexed drawings given by way of example.

### BRIEF DESCRIPTION OF THE DRAWINGS

The invention will be better understood and its various characteristics and advantages will emerge from the following description of a number of exemplary embodiments and its appended figures in which:
- Figure 1 displays the biogenesis of lipid bodies and fused proteins comprising an antibody or fragment thereof or a single-chain variable fragment (scFv) or sdAb with a recombinant protein;
- Figure 2 displays the steps of a particular method of the invention;
- Figure 3 displays an example of a coding sequence for the expression of recombinant proteins fused to scFv by the first genetically engineered organism or part thereof;
- Figure 4 displays an image obtained by phase contrast microscopy and an image obtained with fluorescence microscopy (fluorescein isothiocyanate, FITC-filter) of a mixture comprising oil bodies and a first genetically engineered organism or part thereof comprising a coding sequence for the expression of a recombinant GFP protein fused to an scFv anti-oleosin;
- Figure 5 displays an image obtained by phase contrast microscopy and an image obtained with fluorescence microscopy (using an FITC-filter) of a mix between oil bodies and a GFP proteins;
- Figure 6 displays an image obtained by phase contrast microscopy and an image obtained with fluorescence microscopy (FITC-filter) of oil bodies.

### DETAILED DESCRIPTION OF THE INVENTION

In this specification, the invention will be described by way of examples, demonstrating reduction to practice of the method of the invention. However, the invention is not restricted to these examples.

The present invention relates to a method for the purification of recombinant proteins produced by a first genetically engineered organism, the method comprising the following steps: providing the first genetically engineered organism expressing the recombinant proteins or a part thereof; mixing the first genetically engineered organism or part thereof with oil body proteins which are not obtained from the first engineered organism, wherein the recombinant proteins and/or the oil body proteins are fused with an affinity ligand which allows forming complexes comprising the oil body proteins and the recombinant proteins; and recovering the complexes comprising the oil body proteins and the recombinant proteins by flotation of the mix.

By "first genetically engineered organism or part thereof", it is meant any single living plant, animal, algae, yeast or bacteria which has been genetically engineered. The term "genetically engineered organism" is used here to describe an organism that is chromosomally transgenic, or harbours a plasmid or an autonomous replicating sequence, a recombinant virus or viroid or organisms that express genes transiently without the need for integration into the host genome. The first genetically engineered organism or part thereof may therefore be an organism genetically engineered by introducing nucleic acids for transient expression, or may be an organism obtained from a genetically engineered organism, or the like.

In the case of an animal organism, the cell culture can be a mammalian cell culture (human or non-human) such as mesenchymal stem cells, embryonic stem cells, induced pluripotent stem cells, hematopoietic stem cells, T-lymphocytes (cart-T) or others. In the case of human embryonic stem cells, these are obtained only without destroying the embryo from which they are derived and are not capable of inducing the developmental process of a human being.

By "expressing the recombinant proteins", it is meant that the first genetically engineered organism or part thereof harbours a coding sequence for the expression of a recombinant protein.

By "oil body proteins" it is meant proteins or peptides occurring naturally or partly engineered or fully engineered and which have the capability of targeting their localization to oil bodies, i.e. bodies comprising a lipid core and a phospholipid membranes. Accordingly, any animal, human, bacterial, yeast, fungal or plant oil body protein may be used to perform the method of the invention. Further, according to such a definition of "oil body proteins", the method of the invention also corresponds to the use of only a part of known oil body proteins, native or engineered, as long as such part has the capability of targeting its localization to oil bodies.

By "fused with", it is meant that (1) the first genetically engineered organism or part thereof comprises cells having a nucleic acid construct which includes a coding sequence for a recombinant protein coupled to a coding sequence for an affinity ligand and/or that (2) the oil body proteins are coupled to an affinity ligand. A nucleic acid construct which allows obtaining fusion proteins may comprise coding sequences of proteins which are "linked in a reading frame", i.e. coding sequences which are set on consecutive triplets of DNA, which are non-overlapping.

By "affinity ligand" it is meant a molecule which is able to bind to a specific target, the specific target being according to the invention (1) a recombinant protein and/or (2) an oil body protein, as long as the affinity ligand which is either fused to a recombinant protein or an oil body protein allows forming complexes comprising the oil body protein and the recombinant protein.

By "wherein the recombinant proteins and/or the oil body proteins are fused with an affinity ligand", it is not meant only that multiple recombinant proteins are fused to one unique affinity ligand or that multiple oil body proteins are fused to one unique affinity ligand. One recombinant protein may be fused to affinity ligand and one oil body protein may be fused to one affinity ligand.

By "wherein the recombinant proteins and/or the oil body proteins are fused with an antibody or fragment thereof or a single-chain variable fragment (scFv) or a single-domain antibody (sdAb)", it is meant that multiple recombinant proteins may be fused to one unique antibody or fragment thereof or scFv or sdAb or that multiple oil body proteins may be fused to one unique antibody or fragment thereof or scFv or sdAb. One recombinant protein may be fused to one antibody or fragment thereof or one scFv or one sdAb and one oil body protein may be fused to one antibody or fragment thereof or one scFv or one sdAb.

By « allowing forming complexes » it is meant that (1) the affinity ligands fused with the recombinant proteins are able to target and bind to the oil body proteins mixed, via the affinity ligands, and/or (2) the affinity ligands fused with the oil body proteins are able to target and bind to the recombinant proteins, via affinity ligands. Therefore it is meant that a complex as in the scope of the present invention comprises at least an oil body protein, a recombinant protein and an affinity ligand fused to one of the said oil body protein or said recombinant protein and bound to the other one.

By "a single-chain variable fragment (scFv)", it is meant a fusion protein of the variable regions of heavy and light chains of immunoglobulins, connected with a linker peptide of 10 to 25 amino acids.

By "a single-domain antibody (sdAb)", it is meant an antibody fragment consisting of a single monomeric variable antibody domain, which are also known as camelid, nanobodies or camelid nanobodies.

By "flotation" it is meant that the mix is introduced into a liquid solution, such as a buffer at pH 7, a phosphate-buffered saline solution or the like, and is centrifuged. In the present invention, after this step, the oil bodies, which float to the surface, may be recovered, as they now contain the complexes comprising the oil body proteins and the recombinant proteins.

The method of the present invention comprises mixing the first genetically engineered organism or part thereof with oil body proteins which are not obtained from the first engineered organism. Using oil body proteins which are not obtained from the first engineered organism allows avoiding the need of growing an organism for obtaining the oil body proteins and therefore allows considerably reducing the time required for the production of recombinant proteins which are fully functionalized, notably with the correct protein folding. Notably, a step of growing a genetically engineered organism is a long process which considerably slows the process for obtaining recombinant proteins.

The recombinant protein may be related to proteins used in cosmetic industry, pharmaceutical industry, food industry or agriculture. Advantageously, the recombinant protein is selected among growth factors, interleukins, cytokines enzymes, structural proteins, receptors and signaling proteins, antibodies and antigens.

In the case that the first genetically engineered organism is a plant, the plant is preferably selected among *Nicotiana spp* including *Nicotiana benthamiana.* A genetically engineered plant being a double mutant RdR2 RdR6 of the *Nicotiana benthamiana* species is particularly preferred as first genetically engineered organism. It is particularly advantageous to perform the method with a first genetically engineered organism which is a plant as the proteins are soluble. In addition, providing a plant or part thereof for mixing with oil body proteins may not require any intermediary step of preparation, which allows recovering the recombinant proteins in an easy and fast manner.

Furthermore, in the case that the first genetically engineered organism which is a plant expressing the recombinant proteins has been engineered by introducing a nucleic acid construct comprising the coding sequence of the recombinant proteins via agroinfiltration or other means to induce transient expression of the construct, the expression of the recombinant proteins may be carried out within few days or less and therefore the method of purification of the invention may be performed as soon as the quantity of recombinant proteins expressed and functionalized is considered sufficient. In one embodiment which may be combined with any other embodiment disclosed herein, leaves of a genetically engineered plant are used as they may have been genetically engineered for transient expression. Protein expression in leaves is particularly suited for certain recombinant proteins and allows maintaining a plant growing while carrying out the method on only the leaves of a plant.

In a particular embodiment, which may be combined to any other embodiment wherein the first genetically engineered organism is a plant, the genetically engineered plant expressing the recombinant proteins comprises at least one inactivated gene involved in the Transcriptional Gene Silencing (TGS) and/or at least one inactivated gene involved in the Post Transcriptional Gene Silencing (PTGS) mechanism.

By "gene involved in the Transcriptional Gene Silencing (TGS) and/or Post Transcriptional Gene Silencing (PTGS) mechanism", it is particularly understood as any gene involved in the TGS and/or PTGS mechanism which would prevent the production of the recombinant protein. By "at least one inactivated gene" as used herein it is meant a gene which cannot encode RNA or a protein or encodes no functional protein, or which encodes a protein with reduced activity by at least 90 %. Said "inactivated gene" can encode a protein with reduced functionality, or be a fully inactivated gene, encoding no functional protein or not encoding any protein. An inactivated gene involved in the TGS or PTGS mechanism in the present invention is usually a wild-type gene, i.e. a wild-type gene which, due to the genetically engineered inactivation, cannot encode an RNA or protein, or encodes no functional protein, or encodes a protein with reduced activity, by at least 90 %. A inactivated gene may be obtained by genetic alteration, i.e. at least one among the following modifications: an insertion of a stop codon, an insertion of a nucleotide resulting in the creation of a stop codon, a shift of the nucleotide reading frame resulting in a protein which is not functional, the introduction of a recombination site, such as Cre-Lox, or any modification which has a highly suppressive effect of at least 90% on the expression of the target gene. Other modifications altering the coding sequence of the gene so as to inactivate it out may also be used. Such genetic alterations result from the use of a method selected among CRISPR-Cas 9, synthetic RNAi, TALENs, Meganuclease, TILLING and Zinc finger nuclease but other methods may be suitable. As an alternative, other methods such as physical and chemical mutagenesis by Gamma irradiation or EMS treatment may be used. Preferably, the method used for generating the genetic alterations is CRISPR-Cas9.

Advantageously, the "at least one inactivated gene involved in the TGS or PTGS mechanism" is chosen among the Dicer-like (DCL) family, the Suppressor of Gene Silencing (SGS) family, the Argonaute (AGO) family, the RNA dependent RNA polymerase (RdR) family and combinations thereof.

Advantageously, the "at least one inactivated gene involved in the TGS or PTGS mechanism" includes at least one inactivated gene involved in the TGS mechanism and at least one inactivated gene involved in the PTGS mechanism. For instance, the at least one gene involved in the TGS mechanism may be RDR2 or DCL3 or one of AGOs genes. For instance the at least one gene involved in the PTGS mechanism may be selected from RDR6 or DCL2 or DCL4. Advantageously, the "at least one inactivated gene involved in the TGS and/or PTGS mechanism" includes the RdR2 gene and the RdR6 gene.

The oil body protein means any lipophilic protein capable of anchoring in a lipid body used in the method of the present invention. It may be selected among a list comprising caveolin, oleosin, caleosin, steroleosin, caleosin, perilipins or combinations thereof. Preferably, the oil body protein is an oleosin, as it is, among other things, more conserved through the species.

In the method of the present invention, the step of mixing may use an oilseed plant as it contains more oil body proteins. Preferably, the seeds of an oilseed plant are used for the step of mixing as the seeds typically contain more oil body proteins than other parts of a plant, which would allow forming a greater number of complexes for the recovery of the complexes. Furthermore, for plants which make oilbodies in vegetative structures, including plants engineered for this purpose, non-seed derived oil bodies may be used for the method of the present invention.

In a particular embodiment which may be combined to the other embodiments, the first genetically engineered organism comprises cells comprising a coding sequence for a peptide signal in reading frame with the coding sequence for a recombinant protein and an affinity ligand, such as for instance an antibody or part thereof or a scFv or a sdAb. The PR1b signal peptide allows specifically directing the fused protein which would be expressed from the coding sequence to follow the apoplast pathway, i.e. through the cells rather than the symplast pathway, i.e. through the cytoplasm. In particular, the complexes are less likely to be affected by the proteases in the apoplast pathway, which therefore does not reduce the amount or recombinant protein which may be recovered when the method of the invention is carried out.

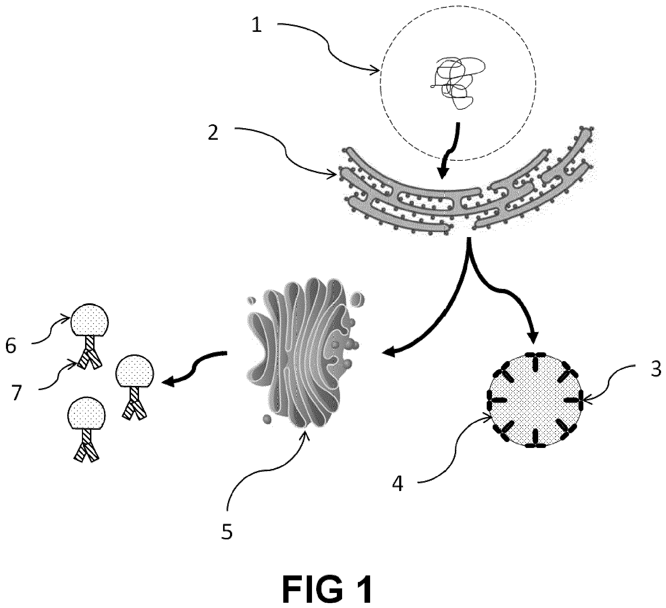

Figure 1 displays the biogenesis of lipid bodies and fused proteins comprising an affinity ligand fused with a recombinant protein. In Figure 1, the nucleus 1 of a cell of the first genetically engineered organism comprises the coding sequence for a recombinant protein in reading frame with an affinity ligand. When expressing such coding sequence, the complete biogenesis for the fused proteins follows the protein biosynthesis process from the nucleus 1, through the endoplasmic reticulum 2 and the Golgi apparatus 5. A fused protein in Figure 1 comprises the affinity ligand 7 and the recombinant protein part 6. Lipid droplets are also produced in cells and comprise lipid bodies which are useful for energy metabolism, membrane synthesis or the production of essential lipid-derived molecules. Oil body proteins are also produced by the cells and are structural proteins which cover lipid droplets.

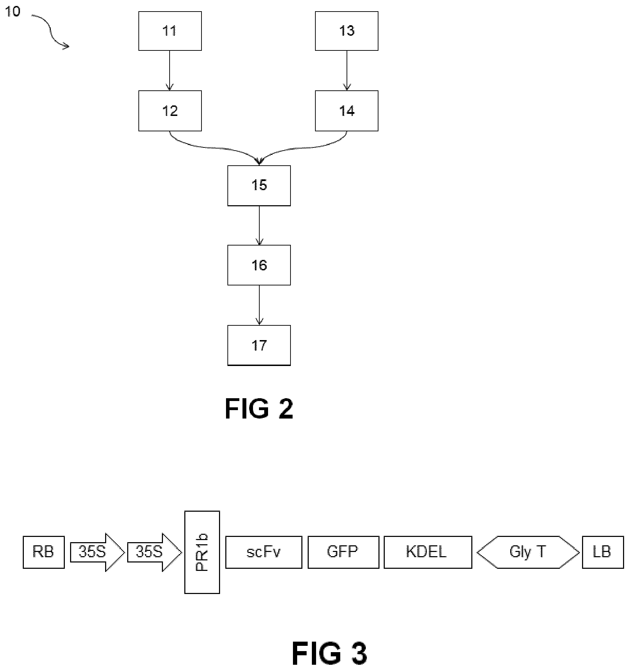

Figure 2 displays a particular method 10 of the invention, comprising providing 11 a first genetically engineered organism being preferably a plant, the first genetically engineered organism expressing fused proteins comprising recombinant proteins fused with antibodies or fragment thereof or scFv or sdAb; preparing 12 the first engineered organism or part thereof for the mixing step 15, for instance by blending a part of the first engineered organism, especially the leaves in the case of a plant; providing 13 a source of oil body proteins which is not obtained from the first genetically engineered organism, such as for example *Camelina sativa* seeds; if necessary passing 14 the source of oil body proteins through a colloid mill or other form of homogenization; mixing 15 the first genetically engineered organism or part thereof with the source of oil body proteins which are not obtained from the first engineered organism; centrifuging 16 the mix obtained at the end of step 15; recovering 17 the supernatant comprising the complexes comprising the oil body proteins and the recombinant proteins. The method may be completed by a further purification of the supernatant to isolate the complexes formed with the oil body proteins, the recombinant proteins and the antibodies or fragment thereof or scFv or sdAb.

Figure 3 displays an example of a coding sequence for the expression of recombinant proteins fused to a scFv by the first genetically engineered organism or part thereof, the coding sequence comprising from the left of the figure to the right: a right border (RB), a tandem 35S promoter, a PR1b signal peptide, an Open Reading Frame encoding a single-chain Fragment variable (scFv) antibody anti oleosin, a GFP marker gene, a KDEL signal retention peptide. Accordingly, and when the first genetically engineered organism comprises such coding sequence, the method of the present invention, and particularly the method as described in the paragraphs describing figure 2, the scFv-GFP fusion protein is produced, for instance in *Nicotiana benthamiana* leaves. These leaves, which may have been blended previously, are then mixed with *Camelina sativa* seeds which may have been milled through a colloid mill or homogenizer previously. The oil body proteins, here oleosins, are recognized by the scFv and therefore bind together, forming complexes of an oil body protein with a scFv which is fused to a recombinant protein being the GFP. Therefore, the oil body proteins on the surface of lipid bodies may be observed by fluorescence.

The PR1b signal peptide (pathogenesis related protein 1) is a signal peptide which allows the recombinant proteins fused to an antibody part thereof or scFv to follow the secretory pathway to the apoplast. In particular, directing the fused proteins allows avoiding the degradation of the fused proteins by proteases which are more present in the cytoplasm or symplast.

The KDEL signal retention peptide is a signal peptide which corresponds to the sequence for the amino acids K-D-E-L. The KDEL signal retention peptide ensures the targeting of the recombinant protein fused to an affinity ligand to the endoplasmic reticulum and retaining it. Further, the KDEL signal retention peptide allows retaining the recombinant protein in the endoplasmic reticulum, which prevents the degradation of proteins and increases their accumulation.

In an example of the present invention, scFv-GFP fusion proteins in *Nicotiana benthamiana* leaves may be produced following agroinfiltration, the scFv targeting oleosins. After the transformed cells of the leaves have expressed the coding sequence for the fused proteins, these leaves may be blended and mixed with *Camelina sativa* seeds. The scFv allows binding with the oleosins and the formation of complexes. The complexes are therefore bound to the oil bodies, allowing the recovery of the recombinant proteins by centrifugation and recovery of the supernatant.

Previous oleosin fusion systems did not allow for the glycosylation of the recombinant proteins of interest because the trafficking of the lipid bodies occurs between the endoplasmic reticulum leaflet and the cytoplasmic membrane. In one aspect of the present invention, this lack of glycosylation is remedied as the antibody or part thereof or scFv is bound to the recombinant protein and follows the secretory pathway through the Golgi apparatus, therefore allowing glycosylation of the recombinant protein of interest.

So as to demonstrate the binding, 50 µL of leaf extract of the following samples: ScFv-GFP + HcPro, GFP + HcPro, were incubated with 100 µL of lipid bodies previously obtained from wildtype seeds and 1ml of Tris-HCI 20mM at pH 7,5 for 3 hours at 28°C under agitation.

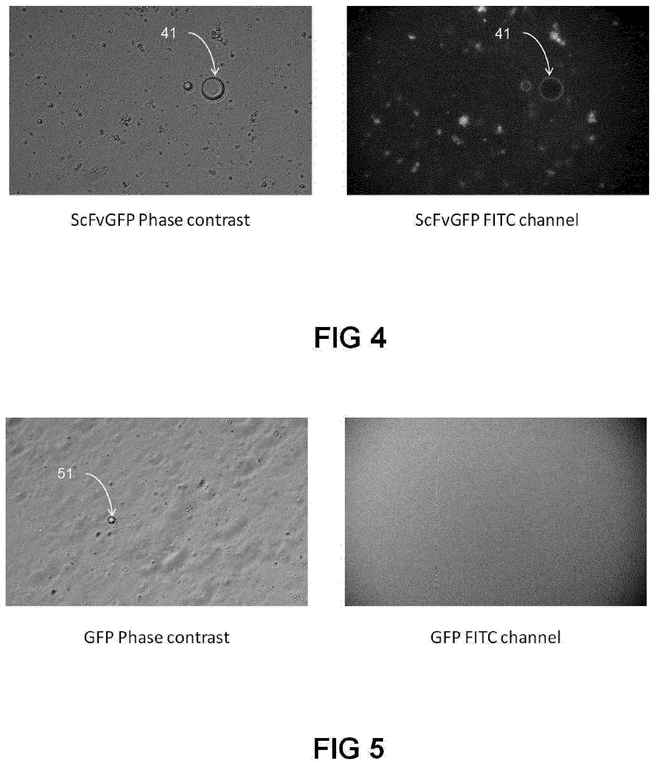

Figure 4 displays an image obtained by phase contrast microscopy and an image obtained with fluorescence microscopy (fluorescein isothiocyanate, FITC-filter) of a mix between oil bodies and a first genetically engineered organism or part thereof comprising a coding sequence for the expression of a recombinant GFP protein fused to a scFv anti-oleosin. Figure 4 displays what can be observed with the example of a coding sequence as described in the paragraph describing figure 3 and with the method as described in the paragraph describing figure 2. On the left part of figure 4, which corresponds to an image obtained by phase contrast microscopy, an oil body 41 can be observed, oil body proteins being present on the surface. On the right part of figure 4, which corresponds to an image obtained by the use of fluorescein isothiocyanate (FITC) filter, the same oil body protein 41 is visible due to the fluorescence of the GFP which is specific to the corona of large lipid bodies and bright spots in lipid bodies. Such microscopy images demonstrate that complexes between the fusion protein comprising the scFv anti-oleosin and GFP and oleosins have been formed.

Figure 5 displays an image obtained by phase contrast microscopy and an image obtained with fluorescence microscopy (fluorescein isothiocyanate, FITC-filter) of a mix between oil bodies and a GFP protein. Figure 5 displays what can be observed when mixing oil bodies with GFP instead of the fusion protein comprising the scFv anti-oleosin and GFP. Lipid body 51 and other smaller lipid bodies can be observed by phase contrast microscopy. However, fluorescence microscopy only reveals any dispersed fluorescence, indicating that no complexes between the oil body proteins and GFP were formed.

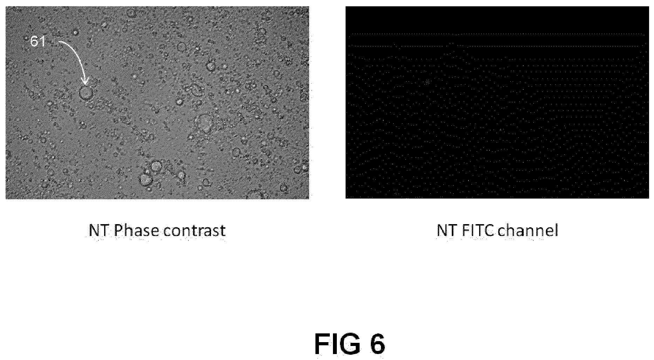

Figure 6 displays an image obtained by phase contrast microscopy and an image obtained with fluorescence microscopy (FITC-filter) of oil bodies. Figure 6 displays oil bodies through phase contrast microscopy and fluorescence microscopy without any GFP or scFv, as a control test. Lipid body 61 can be observed by phase contrast microscopy. However, fluorescence microscopy does not reveal any fluorescence, indicating that the GFP is not present and that the fluorescence observed in figures 5 and 6, regarding fluorescence microscopy, correspond to the GFP.

## Claims

1. A method for the purification of recombinant proteins produced by a first genetically engineered organism, the method comprising the following steps:
- Providing (11) the first genetically engineered organism expressing the recombinant proteins or a part thereof;
- Mixing (15) the first genetically engineered organism or part thereof with oil body proteins which are not obtained from the first engineered organism, wherein the recombinant proteins and/or the oil body proteins are fused with an affinity ligand which allows forming complexes comprising the oil body proteins and the recombinant proteins;
- Recovering (17) the complexes comprising the oil body proteins and the recombinant proteins by flotation of the mix.

2. The method of claim 1, wherein the first genetically engineered organism is a genetically engineered plant.

3. The method of claim 2, wherein providing (11) the first genetically engineered plant expressing the recombinant protein or a part thereof consists in providing the leaves of the genetically engineered plant.

4. The method of one of claims 2 to 3, wherein the genetically engineered plant expressing the recombinant proteins has been engineered by introducing a nucleic acid construct comprising the coding sequence of the recombinant proteins via agroinfiltration.

5. The method of one of claims 2 to 4, wherein the genetically engineered plant or part thereof expressing the recombinant proteins comprises at least one inactivated gene involved in the Transcriptional Gene Silencing (TGS) and/or at least one inactivated gene involved in the Post Transcriptional Gene Silencing (PTGS) mechanism.

6. The method of one of the previous claims, wherein the oil body proteins are selected among a list comprising caveolin, oleosin, caleosin, steroleosin, perilipin or combinations thereof.

7. The method of one of the previous claims, wherein the oil body proteins are oleosin.

8. The method of one of the previous claims, wherein the recombinant proteins are selected among a list comprising insulin, growth factors, therapeutic antibodies, antigens, structural and bioactive peptides or combinations thereof.

9. The method of one of the previous claims, wherein the affinity ligand which allows forming complexes comprising the oil body proteins and the recombinant proteins is selected among: a shark affinity ligand, an artificial affinity ligand derived through phage display or bioinformatics or protein predictive models, or combinations thereof.

10. The method of one of the previous claims, wherein the affinity ligand which is able to form complexes comprising the oil body proteins and the recombinant proteins is selected among: an antibody or fragment thereof, a single-chain variable fragment (scFv), a sdAb (single-domain antibody), or combinations thereof.

11. The method of claim 10, wherein the affinity ligand is a scFv.

12. The method of one of the previous claims, wherein the step of mixing (15) comprises mixing the first genetically engineered organism or part thereof with an oilseed plant comprising oil body proteins, preferably the seeds of an oilseed plant comprising oil body proteins.

13. The method of one of the previous claims, wherein the affinity ligand fused with the recombinant proteins and/or with the oil body proteins contains a cleavage site.

14. The method of one of the previous claims, wherein the method further comprises a step of purifying the recombinant proteins from the complexes.

15. The method according to claim 14, wherein purifying the recombinant proteins from the complexes includes modifying the pH to cleave the affinity ligand from the complexes.
